# EUROPEAN PATENT SPECIFICATION

(11) **EP 0 376 119 B1**
(45) Date of publication and mention of the grant of the patent: **15.06.1994**
(21) Application number: 89123431.2
(22) Date of filing: 19.12.1989
(51) Int. Cl.: C08J 11/06

(54) **A method for recycling PET beverage bottles**
Verfahren zur Wiederverwendung von PET-Getränkeflaschen
Procédé pour recycler des bouteilles de boisson en PET

(30) Priority: 27.12.1988 US 289911; 08.11.1989 IT 6798089
(43) Date of publication of application: 04.07.1990
(73) Proprietor: COBARR S.p.A., I-03012 Anagni Frosinone (IT)
(72) Inventor: Al-Ghatta, Hussain-Ali-Kashif, I-03014 Fiuggi (Frosinone) (IT)
(74) Representative: Rambelli, Paolo

(56) References cited:
- EP-A- 0 227 608
- EP-A- 0 269 583
- FR-A- 2 111 696

## Description

The present invention relates to a method for recycling crushed bottles based on polyethyleneterephthalate and polyethyleneterephthalate copolymers.

The general known method comprises the step of chopping the bottles into small pieces and of separating the PET material from aluminium cap, adhesively bounded paper and polyethylene foot or cup using float-sink techniques or air separation.

Until now, the PET resins are not suitable for reuse as beverage containers because of possible contamination which could interfere with blow molding or with cleanliness required for a food package. Such contamination may derive from an improper use of the bottles in the house-hold to contain liquids (acetone, acetic acid etc.) which diffuse in the bottle walls.

It is known from EP-A-227 608 to treat PET hollow articles in CO₂ under supercritical conditions in order to remove acetaldehyde impurity originated from thermal degradation of PET during the preform injection step.

It is also known from EP-A-269 583 to treat granules of PET with supercritical CO₂ to lower the content of acetaldehyde in order to prevent the sticking of the granules during the step of polycondensation in the solid state.

The object of the present invention is to provide a method for the treatment of crushed bottles from PET resins which makes the treated products suitable for use in food applications.

According to the invention, this object is achieved by virtue of the fact that the crushed bottles are treated in an atmosphere containing carbon dioxide under supercritical conditions.

When recycled PET resins in the forms of crushed bottles are submitted to a supercritical fluid extraction using an atmosphere containing carbon dioxide, the supercritical fluid penetrates the surface of the crushed bottles and extracts the contaminants and impurities out of them.

A mixture of supercritical CO₂ and other supercritical fluids, especially water vapor, could be also used for the purification of the recycled PET.

In order to obtain the best results in the extraction of the impurities, pressures greater than 50 bars and temperatures between 31° and 245°C are preferred.

The invention would be better understood with the aid of the following examples, whose contents should not be understood as limiting of the scope of the present invention.

The impurities contents of the examples have been measured by the head space gas chromatographic method described in EP-A-86830340.5.

### EXAMPLE 1

20 kg of recycled PET crushed bottles, contaminated with acetone to a level of 10.500 ppm, were treated in an autoclave containing CO₂ and 2% by weight of water vapour at 100 bars for 3 hours at an average temperature of 120°C. The gas chromatograph test of the powdered PET after the treatment shows no acetone. The intrinsic viscosity of PET crushed bottles before and after the treatment was respectively 0,787 and 0,778 dl/g.

### EXAMPLE 2

20 kg of recycled PET, contaminated with acetic acid to a level of 20100 ppm, were treated in an autoclave containing CO₂ at 250 bars for 2 hours at an average temperature of 130°C.

The gas chromatographic test shows a content of 3 ppm of acetic acid. There is no decrease in the intrinsic viscosity of the polymer before and after the treatment.

### EXAMPLE 3

20 kg of recycled PET, contaminated with carbon tetrachloride to a level of 10250 ppm, were treated in an autoclave containing CO₂ at 280 bars for 5 hours at an average temperature of 150°C.

The gas chromatographic test of the powdered PET after the treatment shows no carbon tetrachloride.

There is no decrease in the intrinsic viscosity.

### EXAMPLE 4

200 kg of recycled PET, contaminated with trichloroethane to a level of 500 ppm, were treated in an autoclave containing CO₂ at 150 bars for 3 hours at an average temperature of 145°C. The G.C. test shows a content of 2,3 ppm of trichloroethane. There is no decrease of intrinsic viscosity.

### EXAMPLE 5

200 kg of recycled PET, contaminated with methylbenzoate to a level of 220 ppm, were treated in an autoclave containing CO₂ at 165 bars for 5 hours at an average temperature of 155°C.

The G.C. test shows a content of 1,2 ppm of methylbenzoate. There is no decrease of intrinsic viscosity.

Other tests were carried out filling PET bottles with fungicides, insecticides, deodorants, naphta and leaving them on a shelf for one week.

Then the bottles were emptied, crushed and treated according to the present invention. From the recycled PET, new bottles were obtained. These new PET bottles were filled with water that, after a storage of 3 months in the bottles at 40°C has shown no appreciable taste, and it was not possible to detect any of the above impurities in the water.

### EXAMPLE 6

Recycled PET polluted with the products identified in the following table were treated in autoclave containing humified CO₂ (0,5% H₂O) at 130 bars for three hours at an average temperature of 80°C. The test was repeated for each class of pollutant.

For the analysis of the samples, after and before the treatment with CO₂, the polluted PET has been extracted for 24 hours with n-hexane. The effectiveness of the supercritical CO₂ treatment has been determined by gas chromatographic mass spectrometer analysis of the extraction solution. A Perkin Elmer Gas Chromatograph series 8420 with capillary column legth 12m and internal diameter 0,2mm (phase OV1 - Ion Trap Detector - Mass Spectrometer) coupled with NBS/EPA Library of 42.000 Mass Spectra has been used. The results are shown in the following table.

| Analytical results of SFE treatments for decontamination process of recycled PET. | | | | |
|---|---|---|---|---|
| Test No. | Pollution | Contaminant | Before SFE treatment ppm | After SFE treatment ppm |
| 6.1 | A FUNGICIDE | CAPTAN | 5 ppm | ABSENT |
| | | DINOCAP | 5 ppm | ABSENT |
| | | VINCLOZOLIN | 151 | |
| 6.2. | B INSECTICIDE | BENDIOCARB | 13 | ABSENT |
| | | MALATHION | 1 ppm | ABSENT |
| 6.3 | C HERBICIDE | BENTAZON | 139 | ABSENT |
| | | METRIBUZIN | 5 ppm | ABSENT |
| 6.4. | F DRY CLEAN FLUID | ETYLENE TRICHLORO | 19.4 | ABSENT |

### EXAMPLE 7

Recycled PET polluted with household insecticide (type COMBO commercialized by BASF AGRITALIA S.p.A.) were treated in autoclave containing CO₂ at 100 bars for four hours. For the analysis of the sample the same instrumentation of example 6 has been used. Only a qualitative analysis by NBS/EPA Library has been obtained, on which are based the identifications of all the peaks displayed in chromatogram 1 enclosed. The peaks are due to the presence of several compounds which have been identified as the following normal home-insecticide content:
- Peak 1:: DDT PP' 1,1 BIS (4 CHLOROPHENYL) - 2,2,2 - TRICHLOROETHANE
- Peak 2:: DDT 1,1 BIS (4 CHLOROPHENYL) - 2,2,2 - TRICHLOROETHANE
- Peak 3:: P.B.O. PHENOL, 4 (5,6,7,8 TETRAHYDRO - 1,3 - DIOXOLO (4,5,6) ISOQUINOLIN-S-YL) HET
- Peak 4:: NEOPYNAMIN
- Peak 5:: METOXYCHLOR (2,2- BIS (4-METHOXYPHENYL) - 1,1,1 - TRICHLOROETHANE)
The sample treated with supercritical CO₂ shows the chromatogram 2 enclosed. No signal from pesticides is detectable using a higher sensitive monitoring (chromatograms 3.1, 3.2, 3.3 enclosed) instead of the general purpose investigation technique used for the chromatograms 1 and 2.

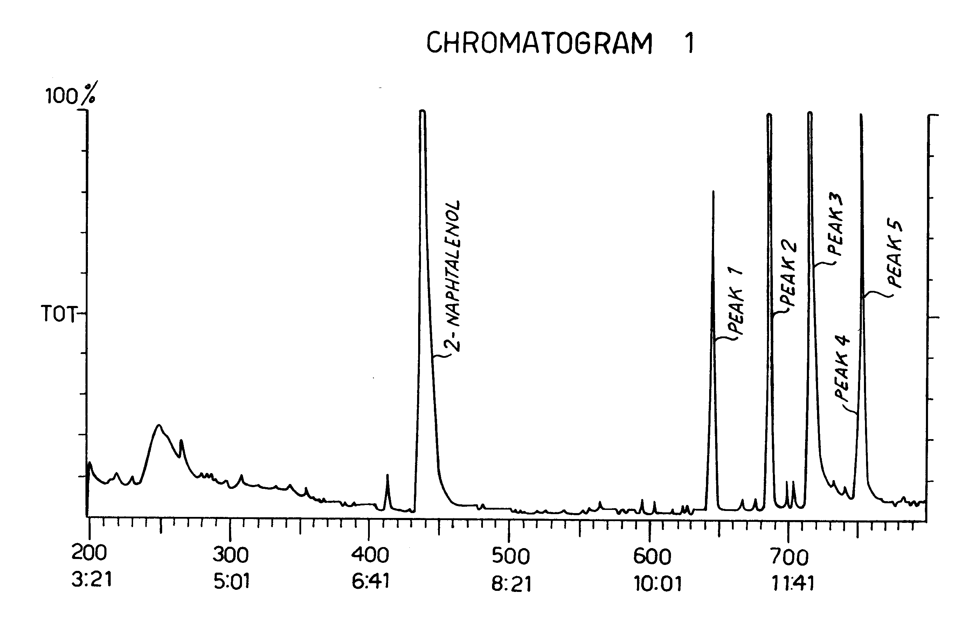

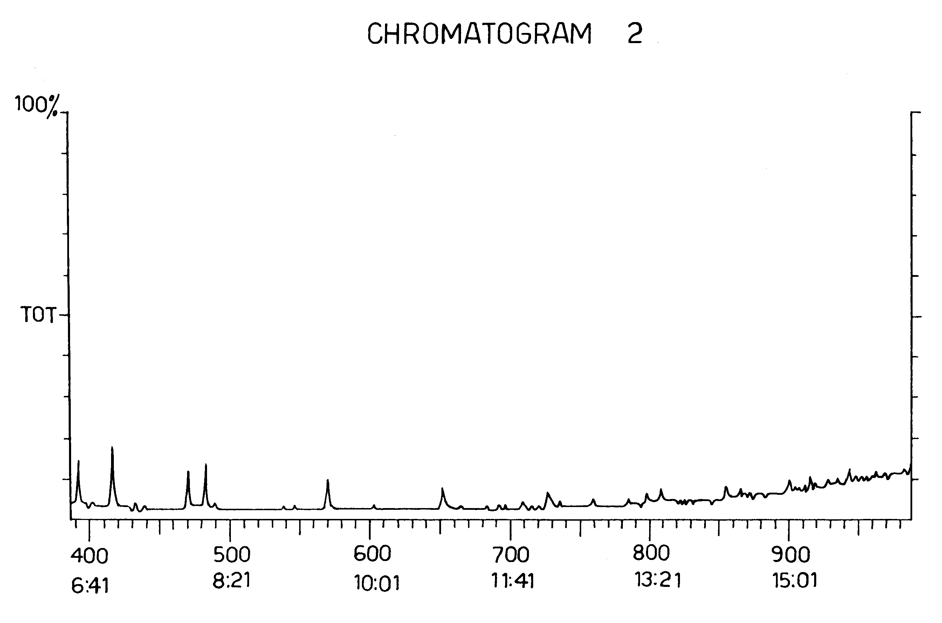

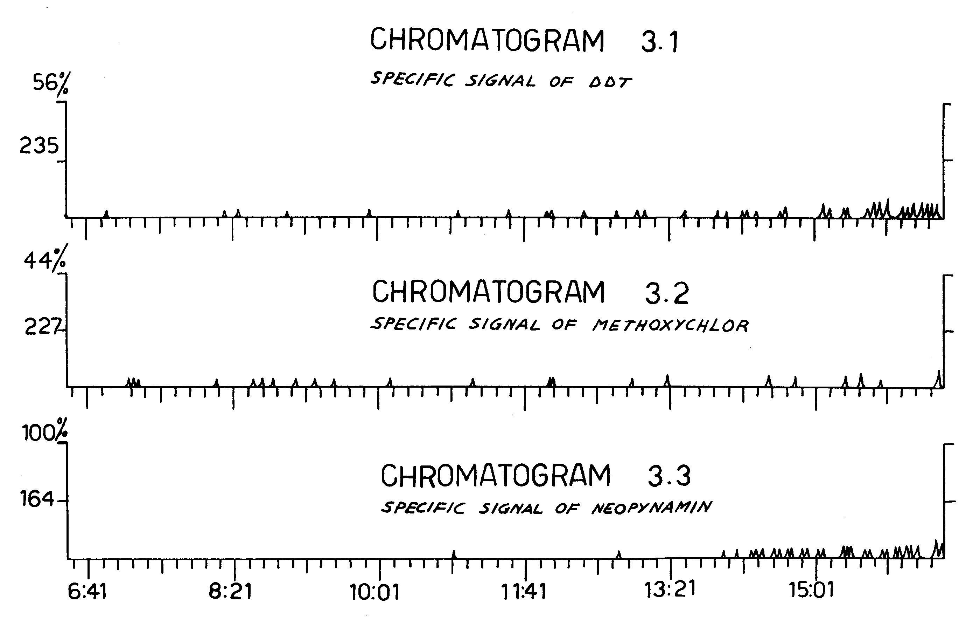

## Claims

1. Method for the treatment of recycled polyethyleneterephthalate resins in the form of crushed bottles containing contaminants different from acetaldehyde, comprising treating the crushed bottles in an atmosphere containing carbon dioxide under supercritical conditions, in order to extract said contaminants out of the resins.

2. A method according to claim 1, wherein the treatment is carried out at a pressure greater than 50 bars.

3. A method according to claim 1, wherein the treatment is carried out at a temperature between 31°C and 245°C.

4. A method according to claim 1, wherein the atmosphere in which the materials are treated contains water vapour in addition to the supercritical carbon dioxide.

## Patentansprüche

1. Verfahren zum Behandeln von zurückgeführten Polyethylenterephthalat-Harzen in der Form von zerkleinerten oder zerstückelten Flaschen, welche Verunreinigungen enthalten, die sich von Acetaldehyd unterscheiden, umfassend die Behandlung der zerstückelten oder zerkleinerten Flaschen in einer Atmosphäre, die Kohlendioxid unter überkritischen Bedingungen enthält, um die genannten Verunreinigungen aus den Harzen zu extrahieren.

2. Ein Verfahren nach Anspruch 1, bei welchem die Behandlung bei einem Druck von größer als 50 bar ausgeführt wird.

3. Ein Verfahren nach Anspruch 1, bei welchem die Behandlung bei einer Temperatur zwischen 31°C und 245°C ausgeführt wird.

4. Ein Verfahren nach Anspruch 1, bei welchem die Atmosphäre, in der die Materialien behandelt werden, zusätzlich zu dem überkritischen Kohlendioxid Wasserdampf enthält.

## Revendications

1. Procédé pour le traitement de résines de poly(téréphtalate d'éthylène) recyclées sous forme de bouteilles écrasées contenant des contaminants différents de l'acétaldéhyde, comprenant l'étape consistant à traiter les bouteilles écrasées dans une atmosphère contenant du dioxyde de carbone dans des conditions supercritiques, afin d'extraire lesdits contaminants des résines.

2. Procédé selon la revendication 1, dans lequel le traitement est effectué à une pression supérieure à 50 bars.

3. Procédé selon la revendication 1, dans lequel le traitement est effectué à une température comprise entre 31°C et 245°C.

4. Procédé selon la revendication 1, dans lequel l'atmosphère, dans laquelle les matières sont traitées, contient de la vapeur d'eau en plus du dioxyde de carbone supercritique.
